# EUROPÄISCHE PATENTSCHRIFT

(11) **EP 2 855 649 B1**
(45) Veröffentlichungstag und Bekanntmachung des Hinweises auf die Patenterteilung: **01.02.2017**
(21) Anmeldenummer: 13727101.1
(22) Anmeldetag: 29.05.2013
(51) Int. Cl.: A61Q 19/10, C11D 1/83, C11D 11/00, C11D 1/66, C11D 1/28, A61K 8/34, A61K 8/41, A61K 8/42, A61K 8/46

(54) **N-METHYL-N-ACYLGLUCAMIN ENTHALTENDE ZUSAMMENSETZUNG**
N-METHYL-N-ACYLGLUCAMINE-CONTAINING COMPOSITION
COMPOSITION CONTENANT DE LA N-MÉTHYL-N-ACYLGLUCAMINE

(30) Priorität: 30.05.2012 DE 102012010660
(43) Veröffentlichungstag der Anmeldung: 08.04.2015
(73) Patentinhaber: Clariant International Ltd, 4132 Muttenz (CH)
(72) Erfinder: KLUG, Peter, 63762 Großostheim (DE); MILDNER, Carina, 65931 Frankfurt am Main (DE)
(74) Vertreter: Holmes, Rosalind
(86) Internationale Anmeldenummer: PCT/EP2013/061105
(87) Internationale Veröffentlichungsnummer: WO 2013/178700

(56) Entgegenhaltungen:
- WO-A1-01/72950
- WO-A1-92/06162
- WO-A1-95/07331
- WO-A1-97/02335
- WO-A2-2010/019841
- DE-A1- 19 508 857
- DE-C1- 4 400 632
- DE-C1- 19 548 068
- US-A- 6 087 320

## Beschreibung

Die Erfindung betrifft ein Tensidkonzentrat, enthaltend mindestens ein anionisches Tensid, ein N-Methyl-N-acylglucamin, ein Lösungsmittel und gegebenenfalls ein oder mehrere Additive, sowie ein Verfahren zur Herstellung des Tensidkonzentrats. Ferner betrifft die Erfindung ein Verfahren zur Herstellung von kosmetischen, dermatologischen oder pharmazeutischen Zusammensetzungen.

EP 0 550 637 B1 beschreibt ein Verfahren zur Herstellung von Polyhydroxyfettsäureamid-Materialien, welche als Tenside u.a. verwendet werden können. Das vorliegende Verfahren ist besonders dann nützlich, wenn das N-Alkylpolyhydroxyamin die Formel N(R¹)CH₂(CH₂OH)₄CH₂OH besitzt. Beim Verfahren bevorzugter verwendeter Fettsäureester-Typ ist ein C₁₂-C₂₀-Fettsäuremethylester. Ein bevorzugtes Verfahren zur Herstellung von Waschmitteltensiden ist eines, bei dem das N-Alkylpolyhydroxyamin ein N-Methylglucamin ist, der Fettsäureester ein C₁₂-C₂₀-Methylester oder eine Mischung davon ist, das Lösungsmittel Methanol und der Katalysator Natriummethoxid ist.

Die obigen Polyhydroxyfettsäureamide haben aber einerseits den Nachteil, dass sie als wasserfreie Tenside hochschmelzende Feststoffe darstellen. Andererseits bilden speziell N-Methyl-N-acylglucamine, die einen C₁₂- und C₁₄-Acylrest oder einen C₁₆- und C₁₈-Acylrest aufweisen, beim Verdünnen in Wasser leicht Gele, die die Handhabung der Produkte stark erschweren.

EP 0 780 464 A2 betrifft ein Verfahren zur Herstellung hellfarbiger, niedrigviskoser Tensidkonzentrate durch Vermischen von Zuckertensiden und Betainen in der Gelphase. Dazu beschreibt EP 0 780 464 A2 ein Verfahren zur Herstellung von hellfarbigen, niedrigviskosen Tensidkonzentraten, bei dem man
(a1) Alkyl- und/oder Alkenyloligoglykoside und/oder
(a2) Fettsäure-N-alkylpolyhydroxyalkylamide und
(b) Betaintenside im Gewichtsverhältnis a : b von 90 : 10 bis 10 : 90 vermischt, mit der Maßgabe, dass die Einsatzstoffe in der Gelphase vorliegen.

Dabei wurde gefunden, dass es für die Herstellung der gewünschten Konzentrate nicht erforderlich ist, von niedrigviskosen, d.h. verdünnten, wässrigen Einsatzstoffen auszugehen und diese nachträglich aufzukonzentrieren. Vielmehr wurde gefunden, dass sich beim Vermischen von konzentrierten Einsatzstoffen, die sich in der Gelphase befinden und somit selbst nicht niedrigviskos sind, Produkte ergeben, die ihrerseits niedrigviskos, hellfarbig und lagerstabil sind. Allerdings beschreibt EP 0 780 464 A2 sowohl keine erfindungsgemäßen Beispiele mit N-Acyl-N-alkylglucamin als auch kein Alkylethersulfat, das nach wie vor das am meisten verwendete und ökonomischste Tensid in Kosmetikmarkt ist.

Die Aufgabe der Erfindung liegt somit darin, ein verbessertes, ökonomisches Tensidkonzentrat bereitzustellen, das die Nachteile des Standes der Technik überwindet, insbesondere ein Konzentrat von einem Tensid bereitzustellen, welches eine verbesserte Handhabung für die Weiterverabeitung bietet.

Demgemäß wird ein Tensidkonzentrat bereitgestellt, enthaltend:
(A) 30-60 Gew.-% mindestens eines anionischen Tensids als Komponente A,
(B) 10-40 Gew.-% mindestens eines N-Methyl-N-acylglucamins als Komponente B, wobei das N-Methyl-N-acylglucamin einen C₈-C₂₂-Acylrest aufweist,
(C) 10-60 Gew.-% mindestens eines Lösungsmittels als Komponente C und
(D) 0-5 Gew.-% eines oder mehrerer Additive als Komponente D, wobei die Summe der Komponenten A bis D 100 Gew.-% ergibt.

Es wurde gefunden, dass die erfindungsgemäßen Tensidkonzentrate eine Viskosität vergleichbar zu marktgängigen Ethersulfatpasten aufweisen und das Tensidkonzentrat beim Verdünnen mit Wasser eine geringere Gelbildung im Vergleich zu Ethersulfatpaste verursacht. Dadurch lassen sich aus den erfindungsgemäßen Tensidkonzentraten hergestellte verdünnte Lösungen im Formulierungsprozess leichter mit weiteren Inhaltsstoffen vermischen und homogenisieren. Die erhaltenen Zusammensetzungen, insbesondere kosmetische, dermatologische oder pharmazeutische Zusammensetzungen, weisen keine Inhomogenitäten auf. Außerdem werden die Rührzeiten bei der Herstellung der Zusammensetzungen verringert.

Vorzugweise weist das erfindungsgemäße Tensidkonzentrat eine Viskosiätbei bei 30°C von 10000-50000 mPas auf, insbesondere eine Viskosität von 15000-30000 mPas.

Weitere Begriffe für N-Methyl-N-acylglucamin sind N-Methyl-N-1-Desoxysorbitol-Fettsäureamid, N-Acyl-N-methyl-glucamin, Glucamid oder N-Methyl-N-alkylglucamid. Dabei entspricht N-Methyl-N-acylglucamin der Formel (X), wobei R ein organischer Rest ist:

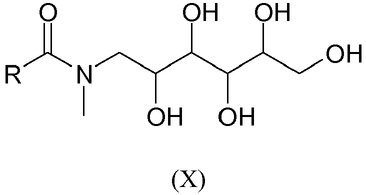

Bevorzugt besteht das Tensidkonzentrat aus
(A) 35-50 Gew.-% mindestens eines anionischen Tensids als Komponente A,
(B) 20-35 Gew.-% mindestens eines N-Methyl-N-acylglucamins als Komponente B, wobei das N-Methyl-N-acylglucamin einen C₈-C₂₂-Acylrest aufweist,
(C) 20-45 Gew.-% mindestens eines Lösungsmittels als Komponente C und
(D) 0-2 Gew.-% eines oder mehrerer Additive als Komponente D, wobei die Summe der Komponenten A bis D 100 Gew.-% ergibt.

Das erfindungsgemäße konzentrierte Tensidkonzentrat erlaubt vorteilhafterweise eine Verdünnung des Konzentrats, obwohl die einzelnene Komponenten, insbesondere N-Methyl-N-acylglucamine, schlecht wasserlösliche Gele bilden. Durch den synergistischen Effekt unter den Komponenten kann vorteilhafterweise diese Eigenschaft erzielt werden.

Im Rahmen einer weiteren bevorzugten Ausführungsform enthält das Tensidkonzentrat
(A) 45-50 Gew.-% mindestens eines anionischen Tensids als Komponente A,
(B) 20-25 Gew.-% mindestens eines N-Methyl-N-acylglucamins als Komponente B, wobei das N-Methyl-N-acylglucamin einen C₈-C₂₂-Acylrest aufweist,
(C) 25-35 Gew.-% mindestens eines Lösungsmittels als Komponente C, und
(D) 0-2 Gew.-% eines oder mehrerer Additive als Komponente D, wobei die Summe der Komponenten A bis D 100 Gew.-% ergibt.

Bevorzugt besteht das Tensidkonzentrat aus den Komponenten A B und C:
(A) 30-60 Gew.-% mindestens eines anionischen Tensids als Komponente A,
(B) 10-40 Gew.-% mindestens eines N-Methyl-N-acylglucamins als Komponente B, wobei das N-Methyl-N-acylglucamin einen C₈-C₂₂-Acylrest aufweist,
(C) 10-60 Gew.-% mindestens eines Lösungsmittels als Komponente C.

Durch die hohe Tensidkonzentration sind die erfindungsgemäßen Zusammensetzungen selbstkonservierend und benötigen keine zusätzlichen Konservierungsmittel.

Im Rahmen einer bevorzugten Ausführungsform ist das Lösungsmittel ein protisches Lösungsmittel.

Im Rahmen einer bevorzugten Ausführungsform besteht das Tensidkonzentrat aus den Komponenten A, B, C, wobei die Komponente C aus Wasser und Propylenglycol oder Wasser, Propylenglykol und Glycerin besteht.

Im Rahmen einer bevorzugten Ausführungsform ist die Komponente A ausgewählt aus einer oder mehreren Verbindung(en) der allgemeinen Formel (I),

R¹SO₃⁻M⁺ (I)

wobei R¹ für Alkyl, Cycloalkyl, Aralkyl, Aryl, Alkoxy, Alkoxyalkyl und Heterocyclyl steht und M⁺ ein Alkalimetall-, Erdalkalimetall- oder ein substituiertes oder nicht substituiertes Ammoniumion ist, oder
der allgemeinen Formel (II),

R¹SO₄⁻ M⁺ (II)

wobei R¹ für Alkyl, Cycloalkyl, Aralkyl, Aryl, Alkoxy, Alkoxyalkyl und Heterocyclyl steht und M⁺ ein Alkalimetall-, Erdalkalimetall- oder ein substituiertes oder nicht substituiertes Ammoniumion ist.

"Alkyl" bedeutet eine gesättigte aliphatische Kohlenwasserstoffgruppe, die geradkettig oder verzweigt sein kann und von 1 bis 20 Kohlenstoffatome in der Kette haben kann. Bevorzugte Alkylgruppen können geradkettig oder verzweigt sein und von 1 bis zu 10 Kohlenstoffatome in der Kette aufweisen. Verzweigt bedeutet, dass eine NiederAlkylgruppe, wie Methyl, Ethyl oder Propyl, an eine lineare Alkylkette angebracht ist. Bei Alkyl handelt es sich beispielsweise um Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, 1-Heptyl, 1-Octyl, 1-Nonyl, 1-Decyl, 1-Undecyl, 1-Dodecyl, 1-Tetradecyl, 1-Hexadecyl und 1-Octadecyl.

"Cycloalkyl" bedeutet einen aliphatischen Ring, der von 3 bis 10 Kohlenstoffatome in dem Ring hat. Bevorzugte Cycloalkylgruppen haben von 4 bis 7 Kohlenstoffatome in dem Ring.

"Aryl" bedeutet Phenyl oder Naphthyl.

"Aralkyl" bedeutet eine Alkylgruppe, die mit einem Arylrest substituiert ist.

"Substituiertes Aralkyl" und "substituiertes Aryl" bedeuten, dass die Arylgruppe oder die Alkylgruppe der Aralkylgruppe mit einem oder mehreren Substituenten ausgewählt aus Alkyl, Alkoxy, Nitro, Carboalkoxy, Cyano, Halo, Alkylmercaptyl, Trihaloalkyl oder Carboxyalkyl substituiert ist.

"Alkoxy" bedeutet eine Alkyl-O-Gruppe, in der "Alkyl" die vorstehend beschriebene Bedeutung hat. Nieder-Alkoxy-Gruppen sind bevorzugt. Beispiele sind Methoxy, Ethoxy, n-Propoxy, i-Propoxy und n-Butoxy.

"Nieder-Alkyl" bedeutet eine Alkylgruppe, die 1 bis 7 Kohlenstoffatome aufweist.

"Alkoxyalkyl" bedeutet eine Alkylgruppe wie vorstehend beschrieben, die mit einer Alkoxygruppe, wie vorstehend beschrieben, substituiert ist. Somit kann unter dem Begriff Alkoxyalkyl ein Polyether verstanden werden.

"Heterocyclyl" bedeutet eine 4 bis 10-gliedrige Ringstruktur, in der ein oder mehrere Ringatome von Kohlenstoff verschieden sind, beispielsweise N, O oder S sind. Heterocyclyl kann aromatisch oder nicht-aromatisch sein, d. h. es kann gesättigt, teilweise oder ganz ungesättigt sein.

Im Rahmen einer bevorzugten Ausführungsform ist das anionische Tensid ausgewählt aus der Gruppe bestehend aus Natriumlaurylethersulfat, Laurylsulfat, Cocosulfat und Mischungen daraus.

Besonders bevorzugt ist Natrium-Cocosulfat oder Natriumlaurylethersulfat.

Im Rahmen einer bevorzugten Ausführungsform ist der Acylrest in der Komponente B aus der Gruppe der linearen oder der verzweigten, der gesättigten oder der ungesättigten C₈-C₂₂-Acylreste und Mischungen daraus ausgewählt.

Im Rahmen einer bevorzugten Ausführungsform besteht die Komponente B aus verschiedenen N-Methyl-N-acylglucaminen, wobei sich die jeweiligen Acylreste unterscheiden und wobei die Acylreste von Carbonsäuren abgeleitet sind ausgewählt aus der Gruppe umfassend Ölsäure, Linolsäure, Caprylsäure, Caprinsäure, Laurinsäure, Myristinsäure, Palmitinsäure und Stearinsäure.

Unter einem Lösungsmittel im Rahmen der Erfindung versteht man vorzugswseise protische Lösungsmittel wie Wasser, C₁-C₈-Alkohole, insbesondere C₁-C₆-Alkohole, Ethylenglykol, Diethylenglykol, Triethylenglykol Propylenglykol, Glycerin oder Mischungen davon, wobei insbesondere Wasser, Wasser und Propylenglykol sowie Wasser und Propylenglykol und Glycerin bevorzugt sind. Von den C₁-C₆-Alkoholen sind Methanol, Ethanol, Isopropanol, n-Butanol oder sek.-Butanol bevorzugt.

Im Rahmen einer bevorzugten Ausführungsform beträgt die Summe der Komponenten A und B von 50 bis 80 Gew.-%, bevorzugt 60 bis 75 Gew.-% und insbesondere 65 bis 70 Gew.-%.

Im Rahmen einer bevorzugten Ausführungsform weist die Komponente B 2 bis 6 verschiedene N-Methyl-N-acylglucamine auf.

Im Rahmen einer bevorzugten Ausführungsform weist die Komponente B von 2 bis 6 verschiedene N-Methyl-N-acylglucamine auf, wobei die 2 bis 6 verschiedenen N-Methyl-N-acylglucamine unterschiedliche geradzahlige Acylreste aufweisen. Das bedeutet beispielsweise, dass die Acylreste von 2 N-Methyl-N-acylglucaminen von der Octansäure oder der Tetradecansäure stammen.

Im Rahmen einer bevorzugten Ausführungsform besteht die Komponente B aus zwei N-Methyl-N-acylglucaminen und die Acylreste unterscheiden sich um höchstens 2 Kohlenstoffatome. Wenn beispielsweise der Acylrest eines N-Methyl-N-acylglucamins ein C₁₆-Acylrest (N-Methyl-N-C₁₆-acylglucamin) ist, dann ist der andere Acylrest ein C₁₈-Acylrest (N-Methyl-N-C₁₈-acylglucamin).

In einer bevorzugten Ausführungsform besteht die Komponente B aus einer Mischung von N-Methyl-N-C₁₂-acylglucamin und N-Methyl-N-C₁₄-acylglucamin.

Im Rahmen einer bevorzugten Ausführungsform sind die Additive aus der Gruppe bestehend aus Komplexierungsmitteln, kationischen Polymeren, Wirkverstärkern, Säuren, Laugen, Konservierungsmitteln, Duftstoffen, Farbstoffen, weiteren Tensiden, Ölkörpern, Überfettungsmitteln, feuchtigkeitsspendenen Mitteln, Stabilisatoren und Mischungen daraus ausgewählt, bevorzugt in Mengen von 0 bis 5,0 Gew.-%, besonders bevorzugt von 0 bis 2,0 Gew.-% und insbesondere von 0,1 bis 1,0 Gew.-%.

Als Konservierungsmittel eignen sich die im betreffenden Annex der europäischen Kosmetikgesetzgebung gelisteten Konservierungsmittel, beispielsweise Phenoxyethanol, Benzylalkohol, Parabene, Benzoesäure und Sorbinsäure, besonders gut geeignet ist beispielsweise 1,3-Bis(hydroxymethyl)-5,5-dimethylimidazolidine-2,4-dione (Nipaguard^{®} DMDMH).

Im Rahmen einer bevorzugten Ausführungsform handelt es sich um ein Konzentrat für kosmetische, dermatologische oder pharmazeutische Zusammensetzungen.

Ein weiterer Gegenstand der Erfindung ist ein Verfahren zur Herstellung des erfindungsgemäßen Konzentrats, umfassend die Schritte:
a) Mischen der Komponenten B und C, wobei vorzugsweise auf 70-90°C erwärmt wird,
b) Zugabe der Mischung aus a) zu Komponene A, wobei vorzugsweise die Temperatur der Komponente A 70-90°C beträgt,
c) Homogenisieren der Mischung aus A, B und C aus c) und
d) gegebenenfalls Zugabe der Komponente E.

Vorzugsweise umfasst das Verfahren die vorstehend genannten Mengenangaben des erfindungsgemäßen Tensidkonzentrats.

Ein weiterer Gegenstand der Erfindung ist ein Verfahren zur Herstellung von kosmetischen, dermatologischen oder pharmazeutischen Zusammensetzungen, wobei das erfindungsgemäße Tensidkonzentrat mit einem Lösungsmittel verdünnt wird. Bevorzugt wird Wasser als Lösungmittel verwendet.

Ein weiterer Gegenstand der Erfindung ist die Verwendung des erfindungsgemäßen Tensidkonzentrats als Reinigungsmittel. Dabei wird das Tensidkonzentrat mit Wasser verdünnt.

Die Erfindung wird durch die nachstehenden Beispiele näher erläutert:
Herstellbeispiele H1 und H2 und Beispiele 1 und 2.

Die im folgenden beschriebene N-Acyl-N-Methylglucamine wurden nach EP 0 550 637 aus den korrespondierenden Fettsäuremethylestern und N-Methylglucamin in Gegenwart von 1,2 Propylenglykol als Lösemittel hergestellt und als Feststoff, bestehend aus Aktivsubstanz und 1,2 Propylenglykol, erhalten.

**Tabelle 1**

| Herstellbeispiel | Methylester | Aktivsubstanz (%) | 1,2-Propylenglykol (%) | Schmelzpunkt |
|---|---|---|---|---|
| H1 | C12/14 | 90 | 10 | 85 |
| H2 | C 16/18 | 80 | 20 | 65 |

Herstellung der erfindungsgemäßen konzentrierten Tensidzusammensetzungen: Die N-Acyl-N-Methylglucamine aus Herstellbeispiel H1 und H2 wurden mit Wasser bei 80 °C verrührt. Die resultierende Lösung wurde in 70 %ige (Natrium)Lauryllethersulfatpaste (Genapol LRO paste), die ebenfalls auf 80 °C erhitzt wurde, eingerührt und homogenisiert.

Zusammensetzung der erfindungsgemäßen Tensidzusammensetzungen:

**Tabelle 2**

| Beispiel | Herstellbeispiel | Laurylethersulfat paste 70 % (%) | Wasser (%) | Aktivgehalt Glucamide | Aktivgehalt Laurylethersulfat |
|---|---|---|---|---|---|
| Beispiel 1 | Herstellbeispiel H1, 25,6 % | 67,1 | 7,3 | 23 | 47 |
| Beispiel 2 | Herstellbeispiel H2, 28,8 % | 67,1 | 4,1 | 23 | 47 |

C12/14 oder C16/18 Glucamid bilden bei Verdünnung normalerweise schlecht wassserlösliche Gele, C12/14 hat aber Vorteile bezüglich der Verdickungsleistung. Die Mischung mit Natriumlaurylethersulfat (SLES) erlaubt leicht verdünnbare Konzentrate mit vorteilhafter Handhabe.

Die erfindungsgemäßen Zusammensetzungen nach Beispiel 1 und 2 sind pumpbare Konzentrate mit vergleichbarer Viskosität zu Laurylethersulfatpaste (Laurylethersulfatpaste (70 %) bei 30°C : 13000 mPa·s; Beispiel 1 bei 30°C : 22000 mPa·s), die sich in Wasser unter Rühren leicht auflösen lassen. Damit wird einerseits die Gelphase des Laurylethersulfats beim Verdünnen reduziert, andererseits die Gelbildung der kettenreinen N-Acyl-N-Methylglucamine in Wasser unterdrückt.

Die Viskositäten wurden mit einem Brookfield-Viskosimeter Model DV II, den Spindeln aus dem Spindelset RV bei 20 Umdrehungen / Minute und 20 °C oder 30 °C gemessen. Es werden die Spindeln 1 bis 7 aus dem Spindelset RV verwendet. Unter diesen Messbedingungen wird Spindel 1 für Viskositäten von maximal 500 mPa·s, Spindel 2 für Viskositäten von maximal 1 000 mPa·s, Spindel 3 für Viskositäten von maximal 5 000 mPa·s, Spindel 4 für Viskositäten von maximal 10 000 mPa·s, Spindel 5 für Viskositäten von maximal 20 000 mPa·s, Spindel 6 für Viskositäten von maximal 50 000 mPa·s und Spindel 7 für Viskositäten von maximal 200 000 mPa·s gewählt.

## Patentansprüche

1. Tensidkonzentrat, enthaltend:
(A) 30-60 Gew.-% mindestens eines anionischen Tensids als Komponente A,
(B) 10-40 Gew.-% mindestens eines N-Methyl-N-acylglucamins als Komponente B, wobei das N-Methyl-N-acylglucamin einen C₈-C₂₂-Acylrest aufweist,
(C) 10-60 Gew.-% mindestens eines Lösungsmittels als Komponente C und
(D) 0-5 Gew.-% eines oder mehrerer Additive als Komponente D, wobei die Summe der Komponenten A bis D 100 Gew.-% ergibt.

2. Tensidkonzentrat nach Anspruch 1, **dadurch gekennzeichnet, dass** das Lösungsmittel ein protisches Lösungsmittel ist.

3. Tensidkonzentrat nach Anspruch 1 oder 2, **dadurch gekennzeichnet, dass** das Tensidkonzentrat aus den Komponenten A, B, C besteht, wobei die Komponente C aus Wasser und Propylenglycol oder Wasser, Propylenglykol und Glycerin besteht.

4. Tensidkonzentrat nach einem der Ansprüche 1 bis 3, **dadurch gekennzeichnet, dass** die Komponente A ausgewählt ist aus einer oder mehreren Verbindung(en) der allgemeinen Formel (I),
R¹SO₃⁻ M⁺ (I)
wobei R¹ für Alkyl, Cycloalkyl, Aralkyl, Aryl, Alkoxy, Alkoxyalkyl und Heterocyclyl steht und M⁺ ein Alkalimetall-, Erdalkalimetall- oder ein substituiertes oder nicht substituiertes Ammoniumion ist, oder
der allgemeinen Formel (II),
R¹SO₄⁻ M⁺ (II)
wobei R¹ für Alkyl, Cycloalkyl, Aralkyl, Aryl, Alkoxy, Alkoxyalkyl und Heterocyclyl steht und M⁺ ein Alkalimetall-, Erdalkalimetall- oder ein substituiertes oder nicht substituiertes Ammoniumion ist.

5. Tensidkonzentrat nach einem der Ansprüche 1 bis 4, **dadurch gekennzeichnet, dass** das anionische Tensid ausgewählt ist aus der Gruppe bestehend aus Natriumlaurylethersulfat, Laurylsulfat, Cocosulfat und Mischungen daraus.

6. Tensidkonzentrat nach einem der Ansprüche 1 bis 5, **dadurch gekennzeichnet, dass** der Acylrest in der Komponente B aus der Gruppe der linearen oder der verzweigten, der gesättigten oder der ungesättigten C₈-C₂₂-Acylreste oder Mischungen daraus ausgewählt ist.

7. Tensidkonzentrat nach Anspruch 6, **dadurch gekennzeichnet, dass** der Acylrest in der Komponente B aus der Gruppe der linearen oder der verzweigten, der ungesättigten C₈-C₂₂-Acylreste oder Mischungen daraus ausgewählt ist.

8. Tensidkonzentrat nach einem der Ansprüche 1 bis 6, **dadurch gekennzeichnet, dass** die Komponente B aus verschiedenen N-Methyl-N-acylglucaminen besteht, wobei sich die jeweiligen Acylreste unterscheiden und wobei die Acylreste von Carbonsäuren abgeleitet sind, ausgewählt aus der Gruppe bestehend aus Ölsäure, Linolsäure, Caprylsäure, Caprinsäure, Laurinsäure, Myristinsäure, Palmitinsäure und Stearinsäure.

9. Tensidkonzentrat nach einem der Ansprüche 1 bis 8, **dadurch gekennzeichnet, dass** die Summe der Komponenten A und B von 50 bis 80 Gew.-% beträgt.

10. Tensidkonzentrat nach einem der Ansprüche 1 bis 9, **dadurch gekennzeichnet, dass** die Komponente B 2 bis 6 verschiedene N-Methyl-N-acylglucamine aufweist.

11. Tensidkonzentrat nach einem der Ansprüche 1 bis 10, **dadurch gekennzeichnet, dass** die Komponente B von 2 bis 6 verschiedene N-Methyl-N-acylglucamine aufweist, wobei die 2 bis 6 verschiedenen N-Methyl-N-acylglucamine unterschiedliche geradzahlige Acylreste aufweisen.

12. Tensidkonzentrat nach einem der Ansprüche 1 bis 11, **dadurch gekennzeichnet, dass** die Komponente B aus zwei N-Methyl-N-acylglucaminen besteht und die Acylreste sich um höchstens 2 Kohlenstoffatome unterscheiden.

13. Tensidkonzentrat nach Anspruch 12, **dadurch gekennzeichnet, dass** wenn der Acylrest eines N-Methyl-N-acylglucamins ein C₁₆-Acylrest (N-Methyl-N-C₁₆-acylglucamin) ist, ist der andere Acylrest ein C₁₈-Acylrest (N-Methyl-N-C₁₈-acylglucamin).

14. Tensidkonzentrat nach einem der Ansprüche 1 bis 13, **dadurch gekennzeichnet, dass** die Additive aus der Gruppe bestehend aus Komplexierungsmitteln, kationischen Polymeren, Wirkverstärkern, Säuren, Laugen, Konservierungsmitteln, Duftstoffen, Farbstoffen, weiteren Tensiden, Ölkörpern, Überfettungsmitteln, feuchtigkeitsspendenen Mitteln, Stabilisatoren und Mischungen daraus ausgewählt sind.

15. Tensidkonzentrat nach einem der Ansprüche 1 bis 13, **dadurch gekennzeichnet, dass** es sich um ein Konzentrat für kosmetische, dermatologische oder pharmazeutische Zusammensetzungen handelt.

16. Verfahren zur Herstellung des Tensidkonzentrats nach einem der Ansprüche 1 bis 14, umfassend die Schritte:
a) Mischen der Komponenten B und C,
b) Zugabe der Mischung aus a) zu Komponene A,
c) Homogenisieren der Mischung aus A, B und C aus c) und
d) gegebenenfalls Zugabe der Komponente E.

17. Verfahren zur Herstellung von kosmetischen, dermatologischen oder pharmazeutischen Zusammensetzungen, **dadurch gekennzeichnet, dass** das Tensidkonzentrat gemäß einem der Ansprüche 1 bis 15 mit einem Lösungsmittel verdünnt wird.

## Claims

1. A surfactant concentrate containing:
(A) 30-60% by weight of at least one anionic surfactant as component A,
(B) 10-40% by weight of at least one N-methyl-N-acylglucamine as component B, where the N-methyl-N-acylglucamine has a C₈-C₂₂-acyl radical,
(C) 10-60% by weight of at least one solvent as component C and
(D) 0-5% by weight of one or more additives as component D, where the sum of the components A to D is 100% by weight.

2. The surfactant concentrate as claimed in claim 1, wherein the solvent is a protic solvent.

3. The surfactant concentrate as claimed in claim 1 or 2, wherein the surfactant concentrate consists of the components A, B, C, where the component C consists of water and propylene glycol or water, propylene glycol and glycerol.

4. The surfactant concentrate as claimed in any one of claims 1 to 3, wherein the component A is selected from among one or more compound(s) of the general formula (I),
R¹SO₃⁻M⁺ (I)
where R¹ is alkyl, cycloalkyl, aralkyl, aryl, alkoxy, alkoxyalkyl or heterocyclyl and M+ is an alkali metal ion, an alkaline earth metal ion or a substituted or unsubstituted ammonium ion, or
of the general formula (II),
R¹SO₄⁻M⁺ (II)
where R¹ is alkyl, cycloalkyl, aralkyl, acryl, alkoxy, alkoxyalkyl or heterocyclyl and M+ is an alkali metal ion, an alkaline earth metal ion or a substituted or unsubstituted ammonium ion.

5. The surfactant concentrate as claimed in any of claims 1 to 4, wherein the anionic surfactant is selected from the group consisting of sodium lauryl ether sulfate, laurylsulfate, cocosulfate and mixtures thereof.

6. The surfactant concentrate as claimed in any of claims 1 to 5, wherein the acyl radical in the component B is selected from the group consisting of linear or branched, saturated or unsaturated C₈-C₂₂-acyl radicals and mixtures thereof.

7. The surfactant concentrate as claimed in claim 6, wherein the acyl radical in the component B is selected from the group consisting of linear or branched, unsaturated C₈-C₂₂-acyl radicals or mixtures thereof .

8. The surfactant concentrate as claimed in any of claims 1 to 6, wherein the component B consists of various N-methyl-N-acylglucamines, where the respective acyl radicals are different and the acyl radicals are derived from carboxylic acids selected from the group consisting of oleic acid, linoleic acid, caprylic acid, capric acid, lauric acid, myristic acid, palmitic acid and stearic acid.

9. The surfactant concentrate as claimed in any of claims 1 to 8, wherein the sum of the components A and B is from 50 to 80% by weight.

10. The surfactant concentrate as claimed in any of claims 1 to 9, wherein the component B comprises from 2 to 6 different N-methyl-N-acylglucamines.

11. The surfactant concentrate as claimed in any of claims 1 to 10, wherein the component B comprises from 2 to 6 different N-methyl-N-acylglucamines, with the 2 to 6 different N-methyl-N-acylglucamines having acyl radicals having different even numbers of carbon atoms.

12. The surfactant concentrate as claimed in any of claims 1 to 11, wherein the component B consists of two N-methyl-N-acylglucamines and the acyl radicals differ by not more than two carbon atoms.

13. The surfactant concentrate as claimed in claim 12, wherein, when the acyl radical of an N-methyl-N-acylglucamine is a C₁₆-acyl radical (N-methyl-N-C₁₆-acylglucamine), the other acyl radical is a C₁₈-acyl radical (N-methyl-N-C₁₈-acylglucamine).

14. The surfactant concentrate as claimed in any of claims 1 to 13, wherein the additives are selected from the group consisting of complexing agents, cationic polymers, activity reinforcers, acids, alkalis, preservatives, fragrances, dyes, further suxfactants, oil bodies oiling agents, moisture-donating agents, stabilizers and mixtures thereof.

15. The surfactant concentrate as claimed in any of claims 1 to 13, wherein it is a concentrate for cosmetic, dermatological or pharmaceutical compositions.

16. A process for producing the surfactant concentrate as claimed in any of claims 1 to 14, which comprises the steps:
a) mixing of the components B and C,
b) addition of the mixture from a) to component A,
c) homogenization of the mixture of A, B and C from c) and
d) if desired addition of the component E.

17. A process for producing cosmetic, dermatological or pharmaceutical compositions, wherein the surfactant concentrate as claimed in any of claims 1 to 15 is diluted with a solvent.

## Revendications

1. Concentré de tensioactif contenant :
(A) 30 à 60 % en poids d'au moins un tensioactif anionique comme composant A,
(B) 10 à 40 % en poids d'une N-méthyl-N-acylglucamine comme composant B, la N-méthyl-N-acylglucamine présentant un résidu acyle en C₈₋₂₂,
(C) 10 à 60 % en poids d'au moins un solvant comme composant C, et
(D) 0 à 5 % en poids d'au moins un additif comme composant D, la somme des composants A à D donnant 100 % en poids.

2. Concentré de tensioactif selon la revendication 1, **caractérisé en ce que** le solvant est un solvant protique.

3. Concentré de tensioactif selon la revendication 1 ou 2, **caractérisé en ce que** le concentré de tensioactif est constitué des composants A, B et C, le composant C étant constitué d'eau et de propylène glycol ou d'eau, de propylène glycol et de glycérine.

4. Concentré de tensioactif selon une des revendications 1 à 3, **caractérisé en ce que** le composant A est choisi parmi un ou plusieurs composés de formule générale (I) :
R¹SO₃⁻ M⁺ (I)
où R¹ représente alkyle, cycloalkyle, arylalkyle, aryle, alcoxy, alcoxyalkyle et hétérocycloalkyle, et M⁺ représente un ion de métal alcalin, de métal alcalino-terreux ou un ion ammonium substitué ou non, ou de formule générale (II) :
R¹SO₄⁻ M⁺ (II)
où R¹ représente alkyle, cycloalkyle, arylalkyle, aryle, alcoxy, alcoxyalkyle et hétérocycloalkyle, et M⁺ représente un ion de métal alcalin, de métal alcalino-terreux ou un ion ammonium substitué ou non.

5. Concentré de tensioactif selon une des revendications 1 à 4, **caractérisé en ce que** le tensioactif anionique est choisi dans le groupe constitué du lauryléthersulfate de sodium, du laurylsulfate, du cocosulfate et de leurs mélanges.

6. Concentré de tensioactif selon une des revendications 1 à 5, **caractérisé en ce que** le résidu acyle du composant B est choisi dans le groupe constitué des résidus acyles en C₈₋₂₂ linéaires ou ramifiés, saturés ou insaturés, ou de leurs mélanges.

7. Concentré de tensioactif selon la revendication 6, **caractérisé en ce que** le résidu acyle du composant B est choisi dans le groupe constitué des résidus acyles en C₈₋₂₂ linéaires ou ramifiés, insaturés, ou de leurs mélanges.

8. Concentré de tensioactif selon une des revendications 1 à 6, **caractérisé en ce que** le composant B est constitué de différentes N-méthyl-N-acylglucamines, dont les résidus acyles sont différents, et dont les résidus acyles dérivent d'acides carboxylique choisis dans le groupe constitué de l'acide oléique, l'acide linoléique, l'acide caprylique, l'acide caprique, l'acide laurique, l'acide myristique, l'acide palmitique et l'acide stéarique.

9. Concentré de tensioactif selon une des revendications 1 à 8, **caractérisé en ce que** la somme des composants A et B est égale à entre 50 et 80 % en poids.

10. Concentré de tensioactif selon une des revendications 1 à 9, **caractérisé en ce que** le composant 3 présente 2 à 6 différentes N-méthyl-N-acylglucamines.

11. Concentré de tensioactif selon une des revendications 1 à 10, **caractérisé en ce que** le composant B présente 2 à 6 différentes N-méthyl-N-acylglucamines, les 2 à 6 différentes N-méthyl-N-acylglucamines présentant des résidus acyles à nombres pairs différents d'atomes de carbone.

12. Concentré de tensioactif selon une des revendications 1 à 11, **caractérisé en ce que** le composant B se compose de 2 N-méthyl-N-acylglucamines et **en ce que** les résidus acyles ont une différence d'au maximum 2 atomes de carbone.

13. Concentré de tensioactif selon la revendication 12, **caractérisé en ce que** si le résidu acyle d'une N-méthyl-N-acylglucamine et une résidu en C₁₆ formant une N-méthyl-N-C₁₆-acylglucamine, l'autre résidu acyle est un résidu en C₁₈ formant une N-méthyl-N-C₁₈-acylglucamine.

14. Concentré de tensioactif selon une des revendications 1 à 13, **caractérisé en ce que** les additifs sont choisis dans le groupe constitué de complexants, de polymères cationiques, d'adjuvants, d'acides, de bases, de conservateurs, d'arômes, de colorants, d'autres tensioactifs, de matières grasses, d'agents surgraissants, d'hydratants, de stabilisants et de leurs mélanges,

15. Concentré de tensioactif selon une des revendications 1 à 13, **caractérisé en ce qu'**il s'agit d'un concentré pour compositions cosmétiques, dermatologiques ou pharmaceutiques.

16. Procédé de fabrication du concentré de tensioactif selon une des revendications 1 à 14, comprenant les étapes suivantes :
a) mélange des composants B et C,
b) ajout du mélange de a) au composant A,
c) homogénéisation du mélange de A, B et C de c) et
d) éventuellement ajout du composant E.

17. Procédé de fabrication de compositions cosmétiques, dermatologiques ou pharmaceutiques, **caractérisé en ce qu'**on dilue le concentré de tensioactif selon une des revendications 1 à 15 avec un solvant.
